# EUROPEAN PATENT APPLICATION

(11) **EP 4 621 792 A1**
(43) Date of publication of application: **24.09.2025**
(21) Application number: 24164511.8
(22) Date of filing: 19.03.2024
(51) Int. Cl.: G16H 20/40, G16H 30/00, G16H 40/63

(54) **DEVICE, SYSTEM AND METHOD FOR PERSONALIZED PREPARATION OF A PATIENT SCHEDULED TO UNDERGO A MEDICAL PROCEDURE**

(71) Applicant: Koninklijke Philips N.V., 5656 AG Eindhoven (NL)
(72) Inventor: SCHADEWALDT, Nicole, Eindhoven (NL); NAUTS, Sanne, Eindhoven (NL); HEUVELINK, Annerieke, Eindhoven (NL); TASAR, Ozgur, Eindhoven (NL)
(74) Representative: Philips Intellectual Property & Standards

(57) **Abstract**

The present invention relates to a device (10), system (100) and method for personalized preparation of a patient scheduled to undergo a medical procedure. The device (10) comprises an input (12) configured to obtain procedural information comprising information about the scheduled medical procedure and physiological information comprising information about the patient's physiological state; a processor (14) configured to construct and execute a personalized preparation program for preparing the patient, and an output (16) configured to output rendering unit signals to a rendering unit (30).

## Description

### FIELD OF THE INVENTION

The present invention relates to device, system and method for personalized preparation, particularly mental preparation, of a patient scheduled to undergo a medical procedure. The medical procedure may generally be any medical imaging procedure (e.g. a medical scan), a medical treatment procedure (e.g. a radiotherapy procedure) or any other diagnostic or interventional procedure.

### BACKGROUND OF THE INVENTION

Medical procedures such as medical image acquisition are often complex and intimidating to the patient. Anxiety can prolong the procedure time, reduce patient compliance, may induce motion (leading to inferior images or results), and healthcare-induced trauma may even hinder further procedures or treatment. Accordingly, the success of procedures is oftentimes related to the cooperation or relaxation of the patient during the procedure: for example, in imaging procedures (radiological procedures such as an MRI-scan or CT-scan), the patient is required to lie still, to follow instructions of how to position themselves, or how to breathe or cooperate in any other way.

The level of cooperation heavily depends on knowledge and understanding of the patient of what kind of cooperation is required from him/her, often including general knowledge of the procedure. The level of cooperation further depends on the acceptance of the patient, that the procedure itself and the cooperation is important for him/her personally. Lastly, the level of cooperation also depends on the nervousness or anxiety of the patient during the procedure, determining how well he/she can concentrate on the procedure and the requirements for cooperation.

Examples of procedures which are often perceived as anxiety-provoking are Magnetic Resonance Imaging (MRI), Computerized Tomography (CT) imaging, X-Ray imaging and interventional procedures. Patient anxiety and lack of cooperation can increase the length of an MR exam and diminish the quality of the acquired MR images.

In many cases, patient information is available to explain the procedure, to prepare them for cooperation and to reduce anxiety. For example, it is regular practice in clinics to inform patients about planned medical procedures. This information includes formal documentation about procedure, risks and potential variations. This formal documentation will in some cases be signed by the patient and a physician. Furthermore, leaflets or similar descriptions about the general procedure are usually given to the patient for self-information. Also, digital preparatory or accompanying material becomes more and more available. Examples for general information material are online videos of procedures.

However, each patient is different, requires a different level of explanation, different reasons for cooperation and different ways to soothe them or reduce anxiety. A skilled nurse or other medical staff member is usually able to talk to the patient, detect their needs, and explain everything in a way suited to that patient. However, the issues preventing optimal preparation in most cases are a lack of availability of skilled nurses or similar personnel and a lack of time availability of the nurse to sufficiently explain the procedure to the patient, in a way that is personalized to that patient's needs.

### SUMMARY OF THE INVENTION

It is an object of the present invention to further improve preparation of a patient scheduled to undergo a medical procedure. At the same time medical staff such as nurses, caregivers or doctors shall be relieved from tasks relating to the preparation of a patient for a medical procedure.

In a first aspect of the present invention a device for personalized preparation of a patient scheduled to undergo a medical procedure is presented, the device comprising:
- an input configured to obtain
   - procedural information comprising information about the scheduled medical procedure; and
   - physiological information comprising information about the patient's physiological state (particularly information about the physiological reactions of the patient during the execution of the (below mentioned) preparation program);
- a processor configured to construct and execute a personalized preparation program for preparing the patient, including
   - - constructing an initial preparation program based on the obtained procedural information;
   - - executing the initial preparation program;

   - obtaining the physiological information indicating the physiological reactions of the patient during the execution of the preparation program;
   - determining mental state information indicating a mental state of the patient during the execution of the preparation program based on the obtained physiological information; and
   - updating the preparation program based on the determined mental state information and generating rendering unit signals for controlling a rendering unit to render visual and/or audible information according to execution of the preparation program; and/or
   - determining, based on the mental state information, whether one or more aspects of the scheduled medical procedure need to be adapted and generating control signals for controlling a control unit to adapt the one or more aspects according to said determination; and
- an output configured to output the rendering unit signals to the rendering unit and, if it is determined that one or more aspects of the medical procedure need to be adapted, to output the control signals to the control unit.

In a further aspect of the present invention a system for personalized preparation of a patient scheduled to undergo a medical procedure is presented, the system comprising:
- a device for preparing a patient scheduled to undergo a medical procedure as presented herein; and
- a rendering unit configured to render visual and/or audible information according to the rendering unit signals obtained from the device.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea to offer a device, system and method capable of preparing a patient scheduled to undergo a medical procedure in a more customized manner. The proposed solution is flexible and adaptive to individual patient needs and allows optimal preparation of patients for a medical procedure such as an MRI or CT scan, or another diagnostic procedure (such as ultrasound, X-ray, fluoroscopy, PET-CT, etc.) or interventional procedure (such as radiotherapy, proton therapy, IGT-procedures, dentistry, vaccination, blood collection, etc.) or any other medical procedure that may be perceived as frightening or stressful by a patient. Generally, the present invention is applicable for any medical procedure where personal coaching (or, in other words, patient preparation) may improve the outcome of the medical procedure.

The preparation program may comprise preparatory and/or guidance material to prepare the patient. For instance, the preparation program may comprise a video or podcast explaining to the patient what will happen during his/her medical procedure. Similarly, the preparation program may comprise a video providing tips for behavior, coaching, reassurance, comfort talk, and/or rules of conduct during the scheduled medical procedure. However, an interactive computer program (e.g., a game) is likewise conceivable to represent an adequate preparation program. In general, there is no requirement for the preparation program to be educational. Accordingly, the preparation program may simply comprise an ambient experience (journey) for the patient to accompany the scheduled medical procedure, the ambient experience comprising soothing video and/or audio content, for example.

In general, the physiological information of the patient, i.e. physiological data/vital parameters of the patient, are obtained during a medical procedure information phase, i.e. during execution of the initial preparation program to deduce relevant properties of the patient during the procedure preparation. The physiological information may then be used to infer the mental state information.

Preferably, the processor is configured to derive/determine (and classify) emotional valence (negative vs. positive) as mental state information. Optionally, more fine-grained ways to categorize a mental state can be used, for example by including arousal (e.g., using the circumplex model of emotion; see Posner, J., Russell, J. A., & Peterson, B. S. (2005): The circumplex model of affect: An integrative approach to affective neuroscience, cognitive development, and psychopathology; Development and psychopathology, 17(3), 715-734).

Using the mental state information the preparation program may be updated and rendering unit signals for controlling a rendering unit to render visual and/or audible information according to execution of the preparation program may be generated. As an alternative (or additionally) to updating the preparation program, it may be determined (based on the mental state information) whether one or more aspects of the scheduled medical procedure need to be adapted and there may be generated (and output) control signals for controlling a control unit to adapt the one or more aspects according to said determination.

Updating the preparation program is preferably performed in real-time.

In general, there are multiple ways to update/adapt and thus (further) personalize (the next steps of) the preparation program or the scheduled medical procedure.

In case there exist aspects of the medical procedure which cannot be changed, such as noise in MR, negative reactions of the patient to the procedure or parts of it may be reduced by providing a preparation program which offers a more detailed explanation of what will happened during the procedure. The patient may become more familiar with the procedure if he/she gets a possibility to replay information about the procedure in several, potentially less frightening, versions. Likewise, the preparation program may be adapted to provide recommendations for workarounds, e.g. the patient may be recommended, that he/she can have earplugs in addition to earmuffs to muffle the noise, for example.

In general, updating/adapting the preparation program may improve patient preparation since physiological reactions of the patient during execution of the preparation program (which includes execution of the initial training program and the updated training program) will be considered for further adapting the preparation program in one or more aspects to the particular patient.

It is assumed that the cooperation and well-being of the patient will be increased by the improved and personalized preparation already, as the patient will be much more aware, of what is needed of him/her.

As a result, by providing excellent preparation, particularly knowledge about what is going to happen during the scheduled medical procedure, the anxiety of the patient will be reduced. The device and method presented herein allows the patient to familiarize herself/himself with unpleasant aspects, can improve cooperation, results, and give a feeling of control for the patient, and thus the implicit retrospective evaluation of it.

However, if aspects of the scheduled medical procedure are perceived negatively and can be changed, the device may likewise adapt one or more of these aspects (directly).

As an example, based on the mental state information (and the procedural information), it may be determined by the processor that the course of the scheduled medical procedure needs to be adapted to ensure that the patient feels comfortable. Accordingly, the processor may generate a control signal for controlling a control unit to adapt, as an aspect of the medical procedure, the course of the medical procedure.

Similarly, it may be determined by the processor that the scheduled medical procedure needs to be re-scheduled. Accordingly, the processor may generate control signals for controlling a control unit to adapt, as an aspect of the medical procedure, the time the medical procedure is scheduled for.

As another example, adapting, i.e. adjusting, a procedure plan as an aspect of the scheduled medical procedure may comprise adjusting breath-hold cycles to a duration that the patient can handle well, for example.

Further, environmental aspects may be taken into account by the device. For example, the lightening in a room may be adapted to the patient's needs as inferred from the mental state information.

In general, aspects of the medical procedure comprise one or more of course of type of medical procedure, duration and/or time of the medical procedure, course of the medical procedure, medical device(s) used in the medical procedure, environment of the medical procedure, particularly room of the medical procedure, medication used in the medical procedure, requirements of the patient during the medical procedure, in particular regarding posture and breathing, travelling time and/or waiting time of the patient before undergoing the medical procedure, requirement of taking medication and/or administering contrast agent, persons accompanying the patient during the scheduled medical procedure, workarounds to better cope with the medical procedure.

The device and method allow personalized preparation for medical procedures in an automatic manner. Optimal procedure execution, optimal procedure results as well as optimal patient evaluation of the procedure, while requiring minimal staff intervention and training, can be achieved using the device and method of the present invention.

In the context of the application, the expressions "scheduled for" and "scheduled to undergo" shall be understood broadly. Generally, someone (e.g. a caregiver, nurse, physician, etc.) tells the patient or plans which medical procedure to undergo so that the patient is scheduled to undergo the medical procedure, most often some longer time in advance. However, even if a patient is not scheduled for a medical procedure long in advance but is coming to an emergency department and has to quickly undergo a medical procedure, one may still want to prepare the patient for the medical procedure in an adaptive and personalized manner. Thus, the expression "scheduled" does not mean that the medical procedure is scheduled a certain minimum time in advance, but generally in any case where it is decided that the patient shall undergo a medical procedure.

According to an embodiment, the processor is configured to construct the initial preparation program by selecting and ordering one or more content components, particularly from a content components database, based on the obtained procedural information.

Thus, already the initial preparation program is adapted to the patient and the planned medical procedure, rather than providing the same general initial preparation program. This helps improving the patient's preparation.

The content components may comprise one or more of video components, image components, spoken and/or written text components, sound components, movie components (particularly, for different medical procedures including the scheduled medical procedure), game components and interactive components.

The processor is preferably configured to select the content components in accordance with (temporal) boundary conditions. In other words, the preparation material provided to the patient should be set up to allow linking the physiological data (i.e. physiological reactions such as facial expressions) and thus mental state information (emotion detection) to parts of the preparation material, i.e. the content components. In particular, the content components presented to the patient should be long enough to allow a patient reaction to develop and to be registered/detected. Furthermore, the order of the content components should be suited to validate the physiological reactions and challenge or reinforce conclusions. Thus, the length of a content component and/or pauses between them should be such that there can be made a differentiation between emotions caused by the components, respectively. In general, the length of the content components depends on the methods / measurements used to obtain the physiological information. While changes in heart rate, for example, may occur rapidly and thus may provide for a high temporal resolution, other measurements, such a galvanic skin response, have delayed occurrence. However, the length of a content component should be at least two seconds, more preferably at least three seconds. Furthermore, between the content components "filler components" may be introduced to get the patient back to a "baseline response", e.g. to lower his/her heart rate, for example.

According to another embodiment, the processor is configured to update the preparation program, based on the mental state information, by adapting the selection and/or order of the one or more content components and/or by adapting one or more of content, level of detail, style of presentation, semantics, words, sounds, and length of the one or more content components.

Updating the preparation program this way may improve patient preparation in accordance with the patient's needs.

For example, if it is deduced from the patient's reaction that a female voice explains the details of the procedures is perceived as less frightening than a male voice, the device may adapt the preparation program accordingly.

In a case where the mental state information is limited, for example, because there were only limited physiological reactions by the patient, the length of the content components may be increased to allow for obtaining more mental state information.

In general, if the mental state information indicates that the patient is feeling well, e.g. if the patient's reaction to the preparation program is "positive", there is no need to intervene, i.e. to adapt the initial training program. However, for content components inducing a "negative" reaction, the upcoming content components could be selected such that the same content component is presented to the patient once more (or repeatedly) to see whether the physiological reaction of the patient changes (positively), i.e. if there is a preparation progress. In some cases, distinguishing between the above-mentioned positive and negative valence may be difficult. In fact, it may be preferable to analyze the patient's level of arousal, particularly a change in the level of arousal. For example, an increase in the patient's heart rate may be a sign of increasing arousal. While such increase may indicate a negative reaction (e.g. fear) it likewise may indicate a positive reaction (e.g. intense happiness). In general, low arousal is often associated with calmness.

If a repetition of the presenting the content component to the patient does not improve his/her mental state (i.e. if valence does not get positive or if the level of arousal does not change/decrease), the processor could be configured to select content components comprising explanatory material. For example, if the patient (repeatedly) reacts negatively to MR noise the patient could be given an (intense) explanation why there is so much noise. Furthermore, there could be provided an explanation what the clinic does to protect the patient from the noise. Role model patients stating that other patients are also afraid of the noise and what helps them could also be comprised in an adapted content component. Furthermore, there could be noise training offered, e.g. giving the patient a slider to play the same noise quietly at first and increase the volume him/herself to a level they can tolerate. This way, they regain some feeling of control, and at the same time get used to the type of noise.

Adapting (the selection and/or order of) the one or more content components may be done based on a look-up table. For instance, the style and/or detail-level may be taken from the look-up table. Alternatively, the preparation program may be re-executed as a kind of trial and error and it may be checked if there is a better patient reaction achieved. Alternatively, artificial intelligence algorithms may be used for this purpose.

According to a preferred embodiment, the processor is configured to assign, as mental state information, a mental state label and/or a mental state score to the one or more content components, the mental state label indicating a type of mental state of the patient during presentation of the respective content component to the patient in the execution of the preparation program and the mental state score indicating a strength of a mental state of the patient during presentation of the respective content component to the patient in the execution of the preparation program, and to update the preparation program based on the mental state label and/or the mental state score.

To ensure creating an appropriate preparation program for the patient, it is relevant to derive reliable and meaningful mental state information. In particular, the processor shall be configured to derive/determine mental state information which allows distinguishing positive and negative emotions and/or different levels of arousal of the patient. In fact, while some physiological information allows to distinguish between low and high levels of arousal (e.g. heart rate, blood pressure, galvanic skin response), other physiological information (e.g. voice information, facial emotion information) may allow for distinguishing between more concrete emotions / valence.

To distinguish between positive and negative emotions or different levels of arousal, the processor may assign mental state labels to the content components the patient is presented. For example, in a case where it is determined from the physiological information that a content component emotionally agitates the patient, said content component may be labelled with a "negative" or "high arousal" label. On the other hand, if it is determined using the physiological information that a content component leads to relaxation of the patient, said content component may be labelled with a "positive" or "low arousal" label. Similarly, a score may be used to label the content components. For example, a content component causing high levels of arousal may be given a higher score than those causing low levels of arousal. The scores may be part of a continuous or a discrete scale. Based on the label or score the processor may then adapt the preparation program, for example by selecting content components which have a score below or above a predetermined score.

In general, the content components may be scored/labelled discretely, e.g. negative/neutral/positive, low/high arousal, or on a continuous scale, e.g. a number from 0 to 10 in accordancewith the patient's behavior/reactions.

Assigning a label or a score to a content component may not be limited to a single content component. In fact, the content components may already be categorized, for example with respect to themes, length etc. Hence, if a patient's reaction to a content component from a particular category is positive, the label "positive" may be assigned to (all) other content components in said category. Likewise, if there are presented several content components from a group of components for the patients, a total group score may be calculated by the processor (e.g. weighted average on the number of themes shown in the group) to determine, which content component(s) (or which group of content components) is best suited to have a positive effect on the patient.

According to another embodiment, the input is configured to obtain additional information, wherein the processor is configured to construct the initial preparation program by selecting and ordering the one or more content components additionally based on the obtained additional information, to update the preparation program additionally based on the additional information and/or to determine, whether one or more aspects of the scheduled medical procedure need to be adapted, additionally based on the obtained additional information, wherein the additional information comprises patient information and/or environment information.

In a preferred embodiment, the input is configured to obtain, as the patient information, information regarding one or more of age, size, weight, gender, health state, medication, medical history, preferences, sensitivity, personality, problems during a prior medical procedure, frequent emotional states, communication preference; particularly wherein the input is configured to obtain the patient information from one or more of a user interface configured to obtain user input, an exam card, a medical record, and a patient information database; and/or the input is configured to obtain, as the environment information, information regarding the room in which the patient will undergo the medical procedure including one or more of devices present in the room, colors in the room, lightening of the room, sound level in the room, persons in the room, layout of the room, ventilation of the room, temperature in the room, humidity in the room, clothes worn by the persons and/or the patient during the medical procedure in the room; wherein the input is configured to obtain the environment information from one or more of a user interface configured to obtain user input, an environment information database, comprising environment information comprising information regarding the room in which the patient will undergo the medical procedure, and an environment sensor configured to measure one or more environmental parameters regarding the room in which the patient will undergo the medical procedure as environment information.

For example, if the room in which the scheduled medical procedure takes place has a low temperature, the processor may select content components suitable for creating a "warm feeling", such as a video of a fireplace or the like. Furthermore, for patients hard of hearing content components comprising subtitles, for example, may be selected.

Similarly, the physiological reactions of the patient to breathing instructions may be observed. During a preparatory video, for example the patient may be asked to follow the instructions, which a demo-patient on the video is doing, and a facial expression tracker may identify, how well the patient is able to follow the instructions. Assuming, that the patient has difficulty to follow, or the facial expression signals nervousness or overwhelmedness, the content components may be adapted such that the breathing instructions are intensified to "train" the patient to follow. At the same time, for example, there may be determined a timespan for which the patient can hold his/her breath as patient information. This patient information may be used to update the preparation program.

According to a further embodiment, the input is configured to obtain, as the physiological information, information regarding any one of heart rate, particularly heart rate variability; respiration rate; blood pressure; change of skin color (as a result of skin perfusion); perfusion of the skin; skin conductance; sweating; facial expressions; eye position or eye movement, particularly pupillary response and/or gaze direction; ; changes in the activation of facial muscles (e.g. the mouth); electroencephalographic (EEG) measurements; electromyographic (EMG) measurements; voice analyses; and/or the input is configured to obtain, as procedural information, information regarding one or more of
- the type of medical procedure,
- the body part of the patient to undergo the medical procedure,
- duration and/or time of the medical procedure,
- course of the medical procedure,
- requirements of the patient during the medical procedure, in particular regarding posture and breathing,
- travelling time and/or waiting time of the patient before undergoing the medical procedure, and
- the requirement of taking medication and/or administering contrast agent.
According to another embodiment, the processor is configured to determine the mental state information from the obtained physiological information by using a database or program relating the physiological information to the mental state information.

For example, facial expressions may be obtained as physiological information, which by the processor may then be related to relevant patient behavior, i.e. to the mental state of the patient.

A slow heart-rate (as physiological information) may generally be linked to low arousal, for example, and may indicate low-arousal emotions such as relaxation or boredom. Furthermore, a slow heart rate may generally be associated with a positive mental state (by the processor).

A fast heart-rate (as physiological information), on the other hand, may indicate nervousness or worry or alertness. If the processer determines, for example, that the heart rate is above a predetermined threshold, the processor may determine that the mental state of the patient is negative.

If the physiological information indicates that the patient's mouth has dropped corners and that there are strong shadows around the mouth, the processor may determine (as the mental state information) that there is a tenseness of the patient. A neutral mouth corner position or lifting slightly upwards and little texture around the mouth, on the other hand, may indicate relaxation, i.e. a positive mental state.

A smile in which only the zygomatic muscle is involved may signal a smile that is communicative, but does not signal joy ("non-Duchenne smile"), whereas a smile that involves both the zygomaticus major and the orbicularis oculi muscle ("Duchenne smile") may signal joy. Emotional expressions can be reliably measured and categorized using known methods, such as "Facial Action Coding System" (FACS; Ekman & Friesen 1978). With FACS, facial expressions are divided into specific "Action Units" (AUs) that are associated with contractions or relaxation of specific facial muscles. Examples of AUs with associated muscular bases are "nose wrinkle" (muscular basis: levator labii superioris alaeque nasi muscle) or "dimpler" (muscular basis: buccinator). These action units are associated with specific emotional expressions.

Furthermore, coding facial expressions can be done automatically using existing methods to detect and categorize emotions, such as "Face Reader" (Lewinski, P., Den Uyl, T. M., & Butler, C. (2014): Automated facial coding: Validation of basic emotions and FACS AUs in FaceReader; Journal of Neuroscience, Psychology, and Economics, 7(4), 227).

Changes in the activation of facial muscles (e.g. the mouth) may either be measured visually (with cameras) or using electromyography (EMG). Using these methods it can be determined which facial muscle is activated or deactivated and with FACS, for example, there is a clear mapping of muscle activation to emotional state.

Gaze can be measured using known eye tracking methods and has been shown to be related to attention and boredom (Kim, J., Seo, J., & Laine, T. H. (2018): Detecting boredom from eye gaze and EEG; Biomedical Signal Processing and Control, 46, 302-313).

Pupillary responses (such as pupil dilation) may be indicative of emotional arousal (e.g. stimuli with negative emotional valence are associated with enlarged pupil size; Kinner, V. L., Kuchinke, L., Dierolf, A. M., Merz, C. J., Otto, T., & Wolf, O. T. (2017): What our eyes tell us about feelings: Tracking pupillary responses during emotion regulation processes; Psychophysiology, 54(4), 508-518).

These and other methods / programs (e.g. based on measuring a person's EMG or EEG) may be used to categorize between different basic emotional expressions such as a neutral expression, happiness, sadness, anger, surprise, fear and disgust, and may provide an index of the extent to which an emotion is displayed on the patient's face. The index may then be used as the score mentioned above.

According to yet another embodiment, the input is configured to obtain the procedural information from one or more of a user interface configured to obtain user input, an exam card (e.g. an exam card for MRI containing information about the MRI sequences to be run), a medical record and a procedural information database and/or the input is configured to obtain the physiological information from one or more of a user interface configured to obtain user input and a patient sensor configured to measure one or more physiological parameters comprising information about the patient's physiological state during execution of the preparation program as physiological information.

In another embodiment of the device, the processor is configured to use the mental state information to determine whether the patient requires extra support, particularly by a doctor or caregiver or by providing medical aids and generating support control signals for controlling a user interface to indicate that the patient requires extra support.

Hence, if it is determined by the device that external help is required, as a fallback strategy, there may be provided a trigger to talk to a nurse, e.g. about an aspect that worries the patient. The nurse might be able to help better and more specific, as she has experience with such situations and the worries of patients.

Furthermore, providing medical aids may further support the patient. In this context the term "medical aids" shall be understood broadly. Medical aids may comprise earplugs, cushions, anti-stress toys or the like, but also medication, particularly tranquilizers.

In an embodiment of the system, the system may further comprise one or more of:
- a control unit (90) configured to adapt one or more aspects of the medical procedure according to the control signals obtained from the device (10);
- a user interface configured to obtain user input of the procedural information, the physiological information, the patient information and/or the environment information;
- a procedural information database comprising procedural information comprising information about medical procedures including the scheduled medical procedure;
- a patient information database comprising patient information comprising information regarding the patient;
- an environment information database comprising environment information comprising information regarding the room in which the patient will undergo the medical procedure;
- a content components database comprising a plurality of content components for constructing a personalized preparation program, in particular comprising one or more of video components, image components, spoken and/or written text components, sound components, movie components for different medical procedures including the scheduled medical procedure, game components;
- one or more patient sensors configured to measure one or more physiological parameters comprising information about the patient's physiological state during execution of the preparation program as physiological information,
   the one or more patient sensors particularly comprising any one of a video camera; a skin conductivity sensor, a heart rate sensor, a blood pressure sensor, a breathing belt;
- one or more environment sensors configured to measure one or more environmental parameters regarding the room in which the patient will undergo the medical procedure as environment information.

Preferably, the system is implemented in a handheld (electronic) device, in particular a smartphone, laptop or tablet, and/or in a computer or a game console; wherein the rendering unit comprises a display configured to display visual information to the patient and/or a loudspeaker to output audible information to the patient.

Preferably the device comprises a camera and/or a microphone. Thus, a smartphone or tablet, i.e. a which is likely equipped with a camera already, is preferred. This way, the technical pre-requisites for capturing facial expressions, for example, to determine mental state information are given.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an implementation of a system according to the present invention;
Fig. 2 shows a schematic diagram of an embodiment of a device according to the present invention;
Fig. 3 shows a schematic diagram of an implementation of a device according to the present invention in the form of a smartphone; and
Fig. 4 shows a schematic diagram of an embodiment of a method according to the present invention.

### DETAILED DESCRIPTION OF EMBODIMENTS

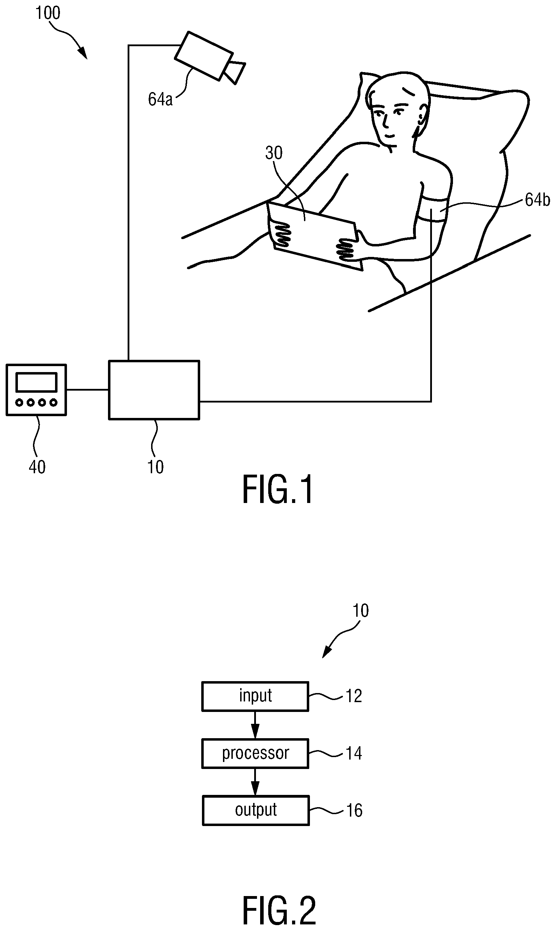

Fig. 1 shows a schematic diagram of an implementation of a system for personalized preparation of a patient scheduled to undergo a medical procedure according to the present invention;

Fig. 2 shows a schematic diagram of an embodiment of a device for personalized preparation of a patient scheduled to undergo a medical procedure according to the present invention;

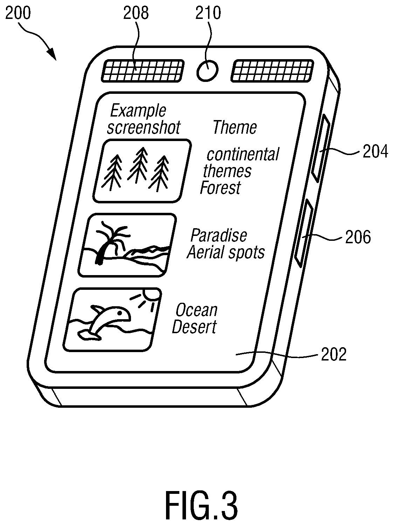

Fig. 3 shows a schematic diagram of an implementation of a device for personalized preparation of a patient scheduled to undergo a medical procedure according to the present invention in the form of a smartphone; and

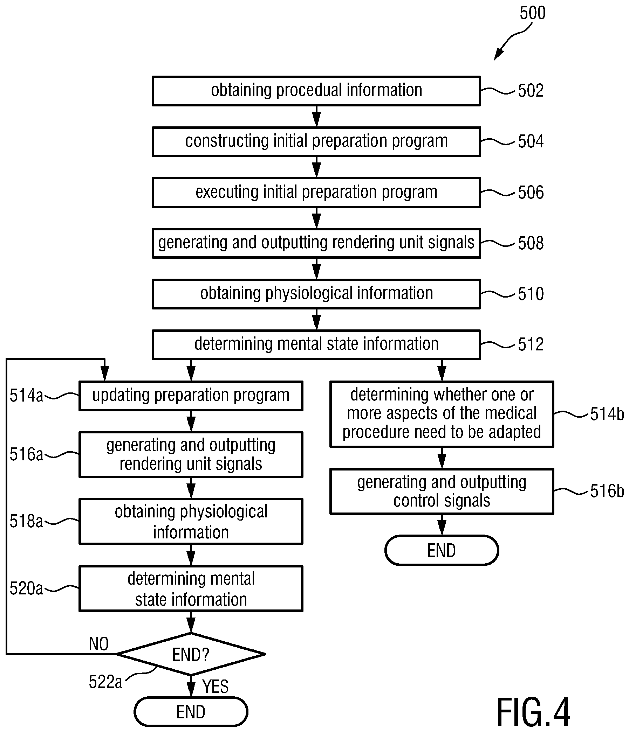

Fig. 4 shows a schematic diagram of an embodiment of a method 500 for personalized preparation of a patient scheduled to undergo a medical procedure according to the present invention.

Fig. 1 shows a schematic diagram of an implementation of a system 100 for personalized preparation of a patient scheduled to undergo a medical procedure. The system 100 comprises a device 10 for personalized preparation of a patient scheduled to undergo a medical procedure as will be explained in more detail below, a rendering unit 30 configured to render visual and/or audible information according to rendering unit signals received from the device 10, a user interface 40 and a first patient sensor 64a and a second patient sensor 64b.

The system 100 may be implemented in a computer or a game console or a handheld device, in particular a smartphone, laptop or tablet.

In the exemplary implementation shown in Fig. 1, a programmed processor represents the device 10, which executes a computer program that is stored in a memory that is accessed by the processor. However, the device 10 may generally be implemented by respective units or circuitry, e.g. a processor, processing circuitry, a computer, dedicated hardware, etc., that carries out the functions of the device 10. Alternatively, a common unit or circuitry, e.g. a common processor or computer, may implement the various functions of the device 10, or separate units or elements may be used that together represent the circuitry.

The rendering unit 30 may generally be any means that outputs visual and/or audible information, e.g. in text form, as image or diagram, as sound or spoken words, etc. In the implementation of Fig. 1 the rendering unit 30 comprises a display configured to display visual information to the patient to output audible information to the patient. In other implementations, the rendering unit 30 may comprise (or be implemented as) a touchscreen, a computer monitor, the screen of a smartphone or tablet, etc. However, loudspeakers are likewise conceivable.

The user interface 40 is configured to receive user input, e.g. of procedural information and additional information (e.g. environmental information). The user interface 40 comprises a keyboard here in this case, however in other implementations the user interface 40 may comprise a touchscreen, microphone and/ or any other entity that allows a user to enter information.

The first patient sensor 64a is implemented as a camera, the camera facing the patient to observe the patient's facial expressions. The second patient sensor 64b is a heart rate sensor configured to measure the heart rate of the patient. Thus, the patient sensors 64a und 64b are configured to measure physiological parameters comprising information about the patient's physiological state during execution of the preparation program as physiological information.

Further patient sensors 64b are conceivable, such as a skin conductivity sensor, a PPG sensor, a blood pressure sensor, a breathing sensor etc.

The system 100 may further comprise a procedural information database 56 containing procedural information comprising information about (medical procedures including) the scheduled medical procedure. The procedural information database 56 may be stored on a server, central storage of a hospital or medical service, in the cloud or at another location that is accessible by the device 10. The procedural information may e.g. be the type of medical procedure (e.g. information about a scan modality (such as MRI or CT), the medical device(s) used in the medical procedure; the body part of the patient to undergo the medical procedure, the duration and/or time of the medical procedure, the course of the medical procedure, e.g. scan sequences.

The system 100 may further comprise a patient information database 52 containing patient information comprising information regarding the patient. The patient information database 52 may be stored on a server, central storage of a hospital or medical service, in the cloud or at another location that is accessible by the device 10.

The system 100 may further comprise an environment information database 54 comprising environment information comprising information regarding the room in which the patient will undergo the medical procedure. The environment information may particularly comprise information regarding the room in which the patient will undergo the medical procedure including one or more of devices present in the room, colors in the room, lightening of the room, sound level in the room, persons in the room, layout of the room, ventilation of the room, temperature in the room, humidity in the room, clothes worn by the persons and/or the patient during the medical procedure in the room.

The system 100 may further comprise a content components database 58 comprising a plurality of content components for constructing a personalized preparation program, in particular comprising one or more of video components, image components, spoken and/or written text components, sound components, movie components for different medical procedures including the scheduled medical procedure, game components, and interactive components. The content components database 58 may be stored on a server, central storage of a content components provider, in the cloud or at another location that is accessible by the device 10. The content components database 58 may be able to generate further content components or provide the content components to another entity for generating further content components.

Two or more of the mentioned databases may also be combined into common databases and/or stored at the same location, such as the cloud or archive or hospital's network.

The system 100 may further comprise one or more environment sensors 62 configured to measure one or more environmental parameters regarding the room in which the patient will undergo the medical procedure as environment information. The environment sensors 62 may comprise one or more of a temperature sensor, a camera, a humidity sensor, a luminosity sensor, etc.

Instead of or in addition to the rendering unit 30 the system may comprise a control unit 90 configured to adapt one or more aspects of the medical procedure according to control signals obtained from the device 10. The control unit 90 may generally be any means that may obtain (i.e. retrieve or receive) control signals and to adapt one or more aspects of the medical procedure in accordance with the control signals. The control unit 90 may e.g. be an environmental control unit configured to control the temperature in a hospital room. However, the control unit may likewise be part of a hospital's scheduling system.

Fig. 2 shows a schematic diagram of a device 10 for personalized preparation of a patient scheduled to undergo a medical procedure. The device 10 comprises an input 12 configured to obtain procedural information comprising information about the scheduled medical procedure and configured to obtain physiological information comprising information about the patient's physiological state and a processor 14 configured to construct and execute a personalized preparation program for preparing the patient, particularly to construct an initial preparation program based on the obtained procedural information, to execute the initial preparation program, to obtain the physiological information indicating the physiological reactions of the patient during the execution of the preparation program, and to determine mental state information indicating a mental state of the patient during the execution of the preparation program based on the obtained physiological information.

The mental state information may then the used by the processor to update the preparation program and to generate rendering unit signals for controlling a rendering unit 30 to render visual and/or audible information according to execution of the preparation program. Alternatively or additionally, the mental state information may be used by the processor to determine whether one or more aspects of the scheduled medical procedure require adaption and to generate control signals for controlling a controlling unit 90 to adapt the one or more aspects according to said determination.

Accordingly, the output 16 is configured to output the rendering unit signals to the rendering unit 30 and/or the control signals to the control unit 90.

The input 12 may be directly coupled or connected to the user interface 40, one or more of the databases 52 to 58, and/or one or more of the environment sensors 62 and/or the patient sensors 64 in order to obtain (i.e. retrieve or receive) the required information. The information may also be stored in a storage, buffer, network, or bus, etc. The input 12 may thus e.g. be a (wired or wireless) communication interface or data interface, such as a Bluetooth interface, Wi-Fi interface, LAN interface, HDMI interface, direct cable connection, or any other suitable interface allowing information transfer to the device 10.

The processor 14 may be any kind of means configured to process the information and determine control signals there from. It may be implemented in software and/or hardware, e.g. as a programmed processor or computer or app on a user device such as a smartphone, smartwatch, tablet, laptop, PC, workstation, etc.

The output 16 may generally be any interface that provides the determined control signals, e.g. transmits them to another device or provides it for retrieval by another device (e.g. a smartphone, computer, tablet, etc.). It may thus generally be any (wired or wireless) communication or data interface.

Preferably, the device 10 is implemented as a handheld device. The term 'handheld device' shall be understood generally as covering any suitable device that is meant to be held by a user when in use, irrespective of whether the patient is actually holding the device or not. The handheld device is preferably a smartphone as this is a device most people own or have direct access to.

As most people own or have access to a suitable handheld device, they can be supplied with the software to run the personalized preparation program on their handheld device. If not, then they may be given a mobile device temporarily to use for the preparation. An advantage is that the patient can do the preparation with the device at a place and time which is convenient for them, for instance at home.

The device 10 may be used to generate preparation material for an upcoming medical procedure, for example video content. Camera-based detection of vital signs, facial expressions and/or emotions, for example, may then be used by the processor to adjust the preparation material, the medical procedure itself, or the information given to the staff.

Fig. 3 shows a schematic diagram of an implementation of a device 10 according to the present invention in the form of a smartphone 200. The smartphone 200 comprises a display 202, an integrated processor 204, a microphone 206, a loudspeaker 208 and a camera 210.

The smartphone 200 may be of any size, shape, model, etc., as long as it is suitable to handle the requirements of being used as a device as disclosed herein.

The display 202 may be of any size or type (e.g. LCD, LED and the like). Displays with a large surface area and the capability of displaying strong colors or small and precise details are preferential. Preferably, the display 202 is integrated in the smartphone 200, but the visual content may also be duplicated or transmitted to an external display connected by wire or wirelessly to the rest of the handheld device, for instance a remote display, goggles or glasses, or any virtual reality device (e.g. virtual reality glasses). An integrated display is the most practical and compact solution, but an external display would still be part of the handheld device, even though that external display may not be meant to be handheld or portable itself. Such an external display may be easier to use in case the patient is not able to watch an integrated display, e.g. due to the patient's position or other reason that prevents the patient from holding the handheld device and watching the integrated display at the same time.

The processor 204 shall be capable of receiving, processing and analyzing incoming information and outputting visual and/or audio information, in real time. The processor 204 may also be in contact with a remote processor, for instance through the internet or through a wired or wireless, such as Bluetooth or Wi-Fi, connection with a local external processor, e.g. a workstation that may support the processor in processing power and/or may provide additional information, such as patient data and/or may store data.

It is preferable that the smartphone 200 is capable of providing audio information, such as sound effects, music, coaching or other informative spoken information and the like. For this purpose, the smartphone is equipped with an integrated loudspeaker 208. Alternatively or simultaneously, a headphone connection may be available to allow a headphone worn by the patient to be connected.

Preferably, the display 202 is a touchscreen and as such does not only serve to display information but also may be used as a user interface to obtain user input, particularly input from the patient.

Using the display 202 a preparation program in the form of a video may be presented to the patient. For example, if the patient shall be prepared for an upcoming MR scan, the patient may be shown video content comprising several video sections, each section (content component) showing different parts of the procedure. For example, a first video section may relate to positioning the patient on the table, a second video section may relate to table movement into the bore, a third video section may relate to MR generated sound, potentially different sound families, e.g. for different sequences, a fourth video section may relate to breathing instructions and a fifth video section may relate to table movement out of the bore.

Using the smartphone 200, a patient's physiological reactions to a preparation program for the upcoming medical procedure can be captured using the camera 210. Furthermore, via the microphone 206 sounds of the patient may be recorded as an additional (or alternative) cue to determine the patient's mental state, particularly emotional valence. In particular, inflection, pitch, and specific verbalizations may be analyzed by the processor to determine the patient's mental state.

Likewise, there could be shown a video show on the display 202 presenting different groups of ambient experience themes with a short explanation, what the group is about e.g. animated photos with small changes in the image and soothing sounds, or videos with relaxing content and little change, or the slide shows offering the most change, as new pictures appear after a short period of time. It should be made clear, if changes in this theme show video indicate a new theme or if they would appear within a theme, e.g. by superimposing theme titles at the start of a new theme.

The patient's reaction to the different themes may then be observed and used to adapt the themes offered on the display 202. In particular, the video may show a selection panel for the different theme groups and patient eye motion may be observed in combination with facial expressions to find out, which group the patient would choose. If the preparatory material is adaptive, the video could continue based on the detected patient gaze (and possibly a (predetermined) theme group score), it could be zoomed in onto the group, that seems interesting for the patient and there could be presented those themes, that were scored highest for this patient. While such a video has not yet a selection function included, it would be very fast and easy for the nurse to select a theme, which the patient is clearly able to describe, e.g. "from the nature videos, I like the dolphins best" or "I would like to see the full calligraphy video in the "story themes" selection".

According to the examples and explanations above the physiological information is provided by the camera 210 and/or the microphone 206. However, in case the display 202 is a touchscreen, physiological data may also be obtained by the touchscreen by measuring the pressure exerted on the touchscreen by the patient when interacting with it. Hence, the measured pressure may be used to determine the mental state information.

The device 10 implemented as a smartphone 200 or other handheld device allows a patient to get familiar with his/her upcoming medical procedure wherever he/she likes, particularly in places where they feel comfortable. Therefore, the device is preferably accessible at home for the patient.

In an example procedure using the device 10, the device 10 may first obtain procedural information via patient input using the display 202, the display 202 being implemented as a touchscreen. The procedural information comprises information about the upcoming medical procedure. In accordance with this information the processor 204 may construct and execute a personalized preparation program for preparing the patient to the scheduled medical procedure. At first, an initial preparation program is constructed based on the obtained procedural information. The initial preparation program may comprise an interactive computer program being shown via the display 202 to the patient. Preferably, the interactive computer program also comprises sound components being presented to the patient via the loudspeaker 208. During execution of the initial preparation program the camera 210 observes the patient's face. Using the camera images the processor 204 determines physiological information indicating the physiological reactions of the patient during the execution of the preparation program. In particular, the patient's skin color, or more precisely change of the skin color, heart rate and facial expressions may be analyzed by the processor 204. Furthermore, gaze tracking may be performed. The processor may relate this information to one or more content components of the preparation program. For example, it may be determined that when content components of the interactive computer program are of low volume, the patient generally smiles. Accordingly, the processor may determine mental state information from the obtained physiological information and update the preparation program based on the determined mental state information. In other words, the deduced reactions of the patient to the preparation program can be used to improve the preparation to achieve a better patient experience and a more successful procedure and cooperation. Of course, also the medical procedure itself may be improved by using the mentioned information.

Fig. 4 shows a schematic diagram of an embodiment of a method 500 according to the present invention. The steps of the method 500 may be carried out by the device 10, wherein the main steps of the method 500 are carried out by the processor 14. The method 500 may e.g. be implemented as computer program running on a computer or processor.

In a first step 502 procedural information comprising information about the scheduled medical procedure may be obtained. The procedural information may comprise information about one or more of type of medical procedure, body part of the patient to undergo the medical procedure, duration and/or time of the medical procedure, course of the medical procedure, requirements of the patient during the medical procedure, in particular regarding posture and breathing, travelling time and/or waiting time of the patient before undergoing the medical procedure, the requirement of taking medication and/or administering contrast agent. Generally, the procedural information can be generated through manual input (e.g. from staff), or it can be taken automatically from medical records (e.g., EMR systems), information systems, or other information sources. These information sources can be combined. The procedural information can be automatically pushed to the device 10, or it can be encoded in a link to an app running on the device 10 (e.g., it can be part of the information provided to patients/their caregivers). For example, patients may receive a (digital) invitation to the medical procedure that includes a QR code containing information about the scheduled procedure.

In a second step 504 and initial preparation program based on the obtained procedural information is constructed. The initial preparation program may comprise video, audio and/or interactive content components.

In a third step 506 the initial preparation program is executed.

In a fourth step 508 rendering unit signals for controlling a rendering unit to render visual and/or audible information according to execution of the initial preparation program are generated and output.

The patient's reactions to the initial preparation program may then be observed, i.e. in a fifth step 510 physiological information comprising information about the patient's physiological state (during the execution of the preparation program) is obtained. In particular, the physiological information indicating the physiological reactions of the patient during the execution of the preparation program.

In the sixth step 512 of the method 500 mental state information indicating a mental state of the patient during the execution of the preparation program is derived/determined based on the obtained physiological information. The mental state information may, for instance, comprise information as to whether the patient reacts positively and or negatively to the preparation program. Such a categorization could be based on a look-up table or on the use of a computer program, possibly supported by artificial intelligence.

In a seventh step 514a the preparation program is updated based on the determined mental state information.

Subsequently, in an eight step 516a rendering unit signals for controlling a rendering unit 30 to render visual and/or audible information according to execution of the preparation program are generated and output to the rendering unit 30.

In step 518a, again, physiological information is obtained and in step 520a, again, mental state information is determined.

In step 522a it is decided whether the method 500 is ended or not. The method may be ended, if the patient decides that the preparation program should be ended (and provides corresponding input), for example. Likewise, the preparation program may be ended once the patient reactions are sufficiently positive. Whether the patient's physiological reactions are sufficiently positive may be decided using a predetermined metric. For instance, if a predetermined number of content components has caused a positive reaction, the preparation program may end. Otherwise, it may be updated further.

Instead of or in addition to steps 514a to 522a, the steps 514b and 516b may be carried out. In step 514b it is determined, based on the mental state information, whether one or more aspects of the scheduled medical procedure require adaption.

In accordance with said determination in step 516b control signals for controlling a control unit 90 are generated and output to the control unit 90. Afterwards, the method 500 is ended. Alternatively, the method may not be ended and step 518a may be carried out.

An example according to the steps illustrated above is given for personalized preparation of Ambient Experience theme content (video, audio, lighting) to accompany an MR examination to reduce patient anxiety, support the patient to lie still and - if necessary - follow instructions.

In this example, a video show of the scheduled MR procedure, Ambient Experience installation with an MR and Ambient Experience themes is shown on a screen with mounted camera to the patient. The camera observes the patient's face during the video display. The input video is processed to determine: a) Skin color change to deduce heart rate; b) eye changes (e.g., changes in pupil dilation, gaze direction) to deduce fear, boredom, fun; and c) mouth changes to deduce fun, relaxation, tension. The processor 14 may then draw the following conclusions: From the initial presentation of the MR procedure, a list of those procedure steps, that are anxiety inducing (fast heart rate, fear in eyes, tension in mouth) is determined. Furthermore, from the presentation of video content, those videos can be selected, where the patient reaction was positive (slow heart rate, smiling eyes, relaxed mouth), and be prioritized according to how positive they were. Accordingly, the following adaptations to the preparation program can be made: First, the MR preparation video can be re-shown to detect, if negative reactions compared to the first presentation were reduced by informing the patient (If not, via a user interface 40 a recommendation can be issued to a medical care person to support in certain steps which are still anxiety inducing). In a more complex embodiment, additional preparatory and anxiety reducing video material could be added for those steps. Secondly, the video display for the actual procedure can still be selected by the patient, but the offered selection includes only videos, which have the best desired effect on the patient. To this enc, a rating/labelling system may be used.

A simpler approach could focus on selection of AE theme videos only based on a video theme show with tracking facial reactions - without the MR procedure component.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device (10) for personalized preparation of a patient scheduled to undergo a medical procedure, the device comprising:
- an input (12) configured to obtain
- procedural information comprising information about the scheduled medical procedure; and
- physiological information comprising information about the patient's physiological state;
- a processor (14) configured to construct and execute a personalized preparation program for preparing the patient, including
- constructing an initial preparation program based on the obtained procedural information;
- executing the initial preparation program;
- obtaining the physiological information indicating the physiological reactions of the patient during the execution of the preparation program;
- determining mental state information indicating a mental state of the patient during the execution of the preparation program based on the obtained physiological information; and
- updating the preparation program based on the determined mental state information and generating rendering unit signals for controlling a rendering unit (30) to render visual and/or audible information according to execution of the preparation program; and/or
- determining, based on the mental state information, whether one or more aspects of the scheduled medical procedure need to be adapted and generating control signals for controlling a control unit (90) to adapt the one or more aspects according to said determination; and
- an output (16) configured to output the rendering unit signals to the rendering unit (30) and, if it is determined that one or more aspects of the medical procedure need to be adapted, to output the control signals to the control unit (90).

2. Device (10) according to claim 1,
wherein the processor (14) is configured to construct the initial preparation program by selecting and ordering one or more content components, particularly from a content components database (58), based on the obtained procedural information.

3. Device (10) according to claim 2,
wherein the processor (14) is configured to update the preparation program, based on the mental state information, by adapting the selection and/or order of the one or more content components and/or by adapting one or more of content, level of detail, style of presentation, semantics, words, sounds, and length of the one or more content components.

4. Device (10) according to claim 3,
wherein the processor (14) is configured to assign, as mental state information, a mental state label and/or a mental state score to the one or more content components, the mental state label indicating a type of mental state of the patient during presentation of the respective content component to the patient in the execution of the preparation program and the mental state score indicating a strength of a mental state of the patient during presentation of the respective content component to the patient in the execution of the preparation program, and to update the preparation program based on the mental state label and/or the mental state score.

5. Device (10) according to any of the preceding claims,
wherein the input (12) is configured to obtain additional information, wherein the processor (14) is configured to construct the initial preparation program by selecting and ordering the one or more content components additionally based on the obtained additional information, to update the preparation program additionally based on the additional information, and/or to determine, whether one or more aspects of the scheduled medical procedure need to be adapted, additionally based on the obtained additional information, wherein the additional information comprises patient information and/or environment information.

6. Device (10) according to claim 5,
wherein the input (12) is configured
to obtain, as the patient information, information regarding one or more of age, size, weight, gender, health state, medication, medical history, preferences, sensitivity, personality, problems during a prior medical procedure, frequent emotional states, communication preference; particularly wherein the input (12) is configured to obtain the patient information from one or more of a user interface (40) configured to obtain user input, an exam card, a medical record, and a patient information database (52);
and/or
to obtain, as the environment information, information regarding the room in which the patient will undergo the medical procedure including one or more of devices present in the room, colors in the room, lightening of the room, sound level in the room, persons in the room, layout of the room, ventilation of the room, temperature in the room, humidity in the room, clothes worn by the persons and/or the patient during the medical procedure in the room; wherein the input (12) is configured to obtain the environment information from one or more of a user interface (40) configured to obtain user input, an environment information database (54), comprising environment information comprising information regarding the room in which the patient will undergo the medical procedure, and an environment sensor (62) configured to measure one or more environmental parameters regarding the room in which the patient will undergo the medical procedure as environment information.

7. Device (10) according to any preceding claim,
wherein the input (12) is configured to obtain, as the physiological information, information regarding any one of heart rate, particularly heart rate variability; respiration rate; blood pressure; change of skin color; perfusion of the skin; skin conductance; sweating; facial expressions; eye position or eye movement, particularly pupillary response and/or gaze direction; changes in the activation of facial muscles; electroencephalographic, EEG, measurements; electromyographic, EMG, measurements; voice analyses;
and/or
wherein the input (12) is configured to obtain, as procedural information, information regarding one or more of
- the type of medical procedure,
- the body part of the patient to undergo the medical procedure,
- duration and/or time of the medical procedure,
- course of the medical procedure,
- requirements of the patient during the medical procedure, in particular regarding posture and breathing,
- travelling time and/or waiting time of the patient before undergoing the medical procedure, and
- the requirement of taking medication and/or administering contrast agent.

8. Device (10) according to any preceding claim,
wherein the processor (14) is configured to determine the mental state information from the obtained physiological information by using a database or program relating the physiological information to the mental state information.

9. Device (10) according to any preceding claim,
wherein the input (12) is configured to obtain the procedural information from one or more of a user interface (40) configured to obtain user input, an exam card, a medical record and a procedural information database (56) and/or wherein the input (12) is configured to obtain the physiological information from one or more of a user interface (40) configured to obtain user input and a patient sensor (64) configured to measure one or more physiological parameters comprising information about the patient's physiological state during execution of the preparation program as physiological information.

10. Device (10) according to any preceding claim,
wherein the processor (14) is configured to use the mental state information to determine whether the patient requires extra support, particularly by a doctor or caregiver or by providing medical aids and generating support control signals for controlling a user interface (40) to indicate that the patient requires extra support.

11. System (100) for personalized preparation of a patient scheduled to undergo a medical procedure, the system (100) comprising:
- a device (10) for preparing a patient scheduled to undergo a medical procedure according to any one of the preceding claims; and
- a rendering unit (30) configured to render visual and/or audible information according to the rendering unit signals obtained from the device (10).

12. System (100) according to claim 11, further comprising one or more of:
- a control unit (90) configured to adapt one or more aspects of the medical procedure according to the control signals obtained from the device (10);
- a user interface (40) configured to obtain user input of the procedural information, the physiological information, the patient information and/or the environment information;
- a procedural information database (56) comprising procedural information comprising information about medical procedures including the scheduled medical procedure;
- a patient information database (52) comprising patient information comprising information regarding the patient;
- an environment information database (54) comprising environment information comprising information regarding the room in which the patient will undergo the medical procedure;
- a content components database (58) comprising a plurality of content components for constructing a personalized preparation program, in particular comprising one or more of video components, image components, spoken and/or written text components, sound components, movie components for different medical procedures including the scheduled medical procedure, game components;
- one or more patient sensors (64) configured to measure one or more physiological parameters comprising information about the patient's physiological state during execution of the preparation program as physiological information,
the one or more patient sensors particularly comprising any one of a video camera; a skin conductivity sensor, a heart rate sensor, a blood pressure sensor, a breathing belt;
- one or more environment sensors (62) configured to measure one or more environmental parameters regarding the room in which the patient will undergo the medical procedure as environment information.

13. System (100) according to claim 11 or 12,
wherein the system (100) is implemented in a handheld device, in particular a smartphone, laptop or tablet, and/or in a computer or a game console;
wherein the rendering unit (30) comprises a display configured to display visual information to the patient and/or a loudspeaker to output audible information to the patient.

14. Method for personalized preparation of a patient scheduled to undergo a medical procedure, the method comprising:
- obtaining procedural information comprising information about the scheduled medical procedure; and
- obtaining physiological information comprising information about the patient's physiological state;
- constructing and executing a personalized preparation program for preparing the patient, including
- constructing an initial preparation program based on the obtained procedural information;
- executing the initial preparation program;
- obtaining the physiological information indicating the physiological reactions of the patient during the execution of the preparation program;
- determining mental state information indicating a mental state of the patient during the execution of the preparation program based on the obtained physiological information;
- updating the preparation program based on the determined mental state information and generating rendering unit signals for controlling a rendering unit (30) to render visual and/or audible information according to execution of the preparation program; and/or
- determining, based on the mental state information, whether one or more aspects of the scheduled medical procedure need to be adapted and generating control signals for controlling a control unit (90) to adapt the one or more aspects according to said determination; and
outputting the rendering unit signals to the rendering unit (30) and, if it is determined that one or more aspects of the scheduled medical procedure need to be adapted, outputting the control signals to the control unit (90).

15. Computer program comprising program code means for causing a computer to carry out the steps of the method as claimed in claim 14 when said computer program is carried out on the computer.
